# EUROPÄISCHE PATENTSCHRIFT

(11) **EP 3 099 700 B1**
(45) Veröffentlichungstag und Bekanntmachung des Hinweises auf die Patenterteilung: **29.08.2018**
(21) Anmeldenummer: 15701497.8
(22) Anmeldetag: 16.01.2015
(51) Int. Cl.: B01J 31/18, C07C 253/00, C07C 45/00, C07C 5/02, C07F 9/6574

(54) **REINIGUNG CHLORVERSCHMUTZTER ORGANOPHOSPHORVERBINDUNGEN**
PURIFYING ORGANOPHOSPHOROUS COMPOUNDS CONTAMINATED WITH CHLORINE
PURIFICATION DE COMPOSÉS ORGANOPHOSPHÉRÉS POLLUÉ PAR DU CHLORE

(30) Priorität: 31.01.2014 DE 102014201756
(43) Veröffentlichungstag der Anmeldung: 07.12.2016
(73) Patentinhaber: Evonik Degussa GmbH, 45128 Essen (DE)
(72) Erfinder: DYBALLA, Katrin Marie, 45657 Recklinghausen (DE); FRANKE, Robert, 45772 Marl (DE); FRIDAG, Dirk, 45721 Haltern am See (DE); PRISKE, Markus, Mobile, Alabama 36609 (US)
(86) Internationale Anmeldenummer: PCT/EP2015/050778
(87) Internationale Veröffentlichungsnummer: WO 2015/113840

(56) Entgegenhaltungen:
- EP-A1- 1 097 936
- EP-A1- 2 003 138
- WO-A1-01/21627
- WO-A1-2012/095255
- US-B1- 6 350 819

## Beschreibung

Die Erfindung betrifft ein Verfahren zur Reinigung eines verunreinigten Organophosphorprodukts, welches mindestens eine Organophosphorverbindung, sowie als Verunreinigung mindestens eine Chlorverbindung enthält. Ferner betrifft die Erfindung ein Verfahren zur Herstellung eines Katalysatorsystems, im Zuge dessen ein verunreinigtes Organophosphorprodukt gereinigt wird, sowie ein Verfahren zur Hydroformylierung oder zur Hydrocyanierung oder zur Hydrierung von ungesättigten Verbindungen in Gegenwart eines homogenen Katalysatorsystems, dessen Ligand auf dieses Weise aufgereinigt wurde.

Organophosphorverbindungen sind chemische Verbindungen, die hauptsächlich die Elemente Kohlenstoff, Wasserstoff und Phosphor enthalten. Optional enthalten sie auch Sauerstoff und Stickstoff. Daneben können die Organophosphorverbindungen weitere Elemente wie Halogene, Schwefel usw. in Form von Substituenten enthalten. Der Begriff umfasst dabei sowohl solche Organophosphorverbindungen, die eine P-C-Bindung aufweisen, als auch solche ohne P-C-Bindung, also mit einer P-O- oder einer P-N-Bindung.

Organophosphorverbindungen haben wegen ihres breiten Anwendungsbereiches eine erhebliche industrielle Bedeutung erlangt. Sie werden unmittelbar als Weichmacher, Flammschutzmittel, UV-Stabilisatoren oder als Antioxidantien eingesetzt. Darüber hinaus stellen sie wichtige Zwischenprodukte bei der Herstellung von Fungiziden, Herbiziden, Insektiziden und Pharmazeutika dar.

Ein spezielles Anwendungsgebiet der Organophosphorverbindungen ist die Katalyse:
So werden insbesondere Phosphine, Phosphite und Phosphoramidite als Liganden in Katalysatorkomplexen verwendet, die wiederum zur homogenen Katalyse von großindustriell betriebenen Prozessen verwendet werden. Dabei ist insbesondere die Hydroformylierung von ungesättigten Verbindungen mit Kohlenmonoxid und Wasserstoff zu nennen, die in der Regel in Gegenwart eines homogenen Katalysatorsystems erfolgt, welches ein Metall sowie mindestens eine Organophosphorverbindung als Ligand aufweist. Eine Einführung in die homogen katalysierte Hydroformylierung bieten:
B. CORNILS, W. A. HERRMANN: Applied Homogeneous Catalysis with Organometallic Compounds. Vol. 1 & 2, VCH, Weinheim, New York, 1996
R. Franke, D. Selent, A. Börner: Applied Hydroformylation. Chem. Rev., 2012, DOI:10.1021/cr3001803

Die Synthese phosphorhaltiger Liganden ist in der Literatur mehrfach beschrieben. Einen guten Überblick findet man in
"Phosphorous(III) Ligands in Homogeneous Catalysis - Design and Synthesis" von Paul C.J. Kamer und Piet W.N.M. van Leeuwen; John Wiley and Sons, 2012.

Bei der Synthese dieser Liganden werden häufig chlorhaltige Reagenzien verwendet. So wird bei der Synthese von Phosphitliganden meist Phosphortrichlorid (PCl₃) eingesetzt.

Die bei der Herstellung von Organophosphorverbindungen eingesetzten Chlorverbindungen bereiten bei der bestimmungsgemäßen Verwendung bzw. Weiterverarbeitung der Organophosphorverbindung vielerlei Schwierigkeiten:
So wird die gewünschte Organophosphorverbindung niemals sofort rein, sondern stets verunreinigt als ein Organophosphorprodukt erhalten, welches neben der gewünschten Organophosphorverbindung auch Verunreinigungen enthält. Bei den Verunreinigungen handelt es sich um nicht oder unvollständig umgesetzten Reagenzien, um Hilfsstoffe oder um Produkte aus Nebenreaktionen. Dabei bereiten insbesondere Verunreinigungen in Form von Chlorverbindungen Schwierigkeiten:
Gelangen die chlorhaltigen Verunreinigungen zusammen mit der als Ligand verwendeten Organophosphorverbindung in einen stählernen Druckreaktor, so ist dieser durch das Chlorid erhöhter Korrosion ausgesetzt. Dies gilt ganz besonders für kontinuierlich betriebene Prozesse, bei denen die Organophosphorverbindungen im Laufe der Reaktion nachdosiert wird. Dies ist beispielsweise bei der Verwendung der Organophosphorverbindung als Ligand in der großtechnischen Hydroformylierung der Fall. Durch die Nachdosierung kommt es zwangsläufig auch zu einer Akkumulation der Nebenkomponenten im Reaktor. Dies ist insbesondere dann kritisch, wenn Chlorid eine der Nebenkomponten darstellt, da Chlorid selbst rostfreie Stähle angreift:
Merkblatt 893 "Edelstahl rostfrei für die Wasserwirtschaft", 1. Auflage 2007, Hrsg. Informationsstelle Edelstahl Rostfrei, Düsseldorf.

In Anwesenheit von Chloridionen droht insbesondere eine Spannungsrisskorrosion, welche im günstigeren Fall zu einer vorzeitigen Abstellung des Prozesses und zur Überholung des Reaktors führt, im ungünstigsten Fall aber auch ein Bersten des Reaktors zur Folge haben kann. Es ist daher von eminenter Bedeutung, ein Einschleppen von chlorhaltigen Verbindungen über das organophosphorhaltige Katalysatorsystem zu unterbinden.

Die einsatzbereiten phosphorhaltigen Liganden sollte weniger als 10 000 ppm, besser noch weniger als 1 000 ppm Gesamtchlor enthalten. Bei einem Gesamtchlorgehalt in dieser Größenordnung ist in technisch ausgeführten Prozessen die Gefahr von Spannungsrisskorrosion im Reaktor beherrschbar. Zwar sieht das oben zitierte Merkblatt bereits einen Chlorgehalt von 200 ppm als kritisch an, jedoch wird in industriellen chemischen Prozessen die Organophosphorverbindung lediglich in katalytischen Mengen eingesetzt, sodass der Gesamtchlorgehalt im Reaktor aufgrund der Verdünnung durch die Reaktionsteilnehmer deutlich unter 200 ppm beträgt, wenn der Verunreinigungsgrad der eingesetzten Liganden im angestrebten Bereich liegt.

Der Chloridgehalt lässt sich analytisch einfach bestimmen; zum Beispiel durch wässrige Titration.

Weitreichender ist die Bestimmung des Gesamtchlorgehalts, der neben den Chloriden auch anderweitig gebundenes Chlor umfasst. Ein Abstellen auf den Gesamtchlorgehalt ist auch insoweit sachdienlich, als nicht ausgeschlossen werden kann, dass auch anderweitig gebundenes Chlor den Reaktor zu schädigen vermag. Bei der Bemessung der Grenzwerte für Gesamtchlor bleibt aber der Chloridanteil maßgeblich.

Ein geeignetes Verfahren zur Bestimmung des Gesamtchlorgehalts ist die Verbrennung nach Wickbold mit Probenvorbereitung nach DIN 51408 und Messung per lonenchromatographie nach DIN EN ISO 10304.

In der Patentliteratur sind verschiedene Methoden zur Reduktion des Gesamtchlorgehalts von Organophosphor-Liganden in Anschluss an die eigentliche Synthese bekannt:
EP 0 285 136 offenbart ein Verfahren zur Reinigung von tertiären Organophosphiten von fünfwertigen Organophosphor-Verbindungen, die als Nebenprodukte der Synthese oder auch als Abbau- bzw. Hydrolyseprodukte der tertiären Organophosphite entstehen. Das Verfahren sieht eine Behandlung des gelösten verunreinigten Organophosphits mit Wasser bei erhöhter Temperatur in Gegenwart einer Lewis-Base vor. Als Lewis-Basen werden anorganische Salze (Carbonate, Hydroxide, Oxide), tertiäre Amine und Polymere, die Amin-Gruppen tragen, eingesetzt.

Ein Nachteil dieses Verfahrens liegt in der Behandlung mit Wasser. Nicht nur die zu entfernenden Verunreinigungen sondern auch die tertiären Organophosphite selbst reagieren unter den genannten Bedingungen, so dass, je nach Hydrolysestabilität der Organophosphite, ein Teil des Wertprodukts verloren geht.

DE 10 2004 049 339 beschreibt ein Verfahren zur Reinigung von phsophorhaltigen Chelatliganden mittels Extraktion unter Verwendung eines polaren Extraktionsmittels. Der Rohligand wurde hierbei sechsmal mit einem polaren Lösungsmittel extrahiert und weist im Anschluss einen Gehalt an Aminbase, Aminhydrochlorid oder deren Gemischen von weniger als 100 ppm auf. Bei dieser Art der Aufreinigung sind jedoch enorme Mengen an Lösungsmittel notwenig, was aus ökonomischer und ökologischer Sicht verbesserungswürdig ist.

Aus CN 101684130 A ist die Aufreinigung von Phosphitliganden durch den Zusatz von entionisiertem Wasser und der anschließenden Extraktion bekannt. Das organische Lösungsmittel wird in einem folgenden Schritt destillativ entfernt und das Rohprodukt erneut umkristallisiert. Auf diesem Wege konnte ein Produkt mit einem Rest-Chlorgehalt von 0,01 Gewichts-% Chlor erhalten werden.

Um mit dieser Methode den Chlorgehalt des Liganden zu reduzieren, sind somit eine Extraktion und eine anschließende Umkristallisation notwendig. Das bedeutet, dass viel Lösungsmittel eingesetzt werden muss und Ausbeuteverluste aufgrund der verschiedenen Reinigungsschritte und der ggf. nicht vorhandenen Hydrolysestabilität der Organophosphite ein Teil des Wertprodukts verloren geht.

In WO 2012 095253 wird ein Verfahren zur Herstellung von 6,6'-[(3,3'-Di-tert-butyl-5,5'-dimethoxy-1,1'-biphenyl-2,2'diyl)bis(oxy)]bis(dibenzo[d,f][1,3,2]dioxaphosphepin (genannt: "Biphephos") beschrieben. Die Reinigung des Liganden erfolgt durch mehrmaliges Waschen mit verschiedenen Lösungsmitteln.

Neben Waschen, Destillieren und Umkristallisation besteht im Zuge der Herstellung von Organophosphorverbindungen auch die Möglichkeit, das verunreinigte Organophosphorprodukt mit Hilfe von Filtration zu reinigen:
So beschreibt EP2091958B1 die Herstellung von Bisphosphiten, welche in Toluol gelöst erhalten und filtriert werden. Dabei kommt es lediglich zu einer teilweisen Entfernung des Chlors, was in Hinblick auf die beabsichtigte Verwendung der hergestellten Organophosphorverbindung als Ligand in der Hydroformylierung von Olefinen verbesserungsbedürftig ist.

Aus der EP1097936B1 ist es bekannt, ein in einem organischen Lösungsmittel gelöstes, als Stabilisator bestimmtes Organophosphorprodukt zu reinigen, indem der Lösung Wasser, eine Base und ein festes Trocknungsmittel zur Trocknung der organischen Phase zugegeben werden. Dabei entstehen zwei Schichten, nämlich eine wässrige und eine organische. Die beiden Schichten werden getrennt, das Trocknungsmittel wird aus der organischen Phase abfiltriert und das Lösungsmittel verdampft. Die chlorhaltigen Verunreinigungen werden also über die wässrige Phase abgeführt. Der Nachteil dieses Verfahrens ist in der Zugabe des Wassers zu sehen, denn die Abtrennung der wässrigen Phase wird in der Produktion im industriellen Maßstab nicht mit der erforderlichen Reinheit gelingen, sodass Restwasser in dem gereinigten Produkt zurück bleibt. Dort vermag es durch Hydrolyse die soeben gewonnene Organophosphorverbindung wieder zu zersetzen. Insbesondere dann, wenn das Organophosphorprodukt für die Verwendung als Ligand in Katalysatorkomplexen bestimmt ist, ist die Anwesenheit von Wasser in Hinblick auf die Hydrolyse abzulehnen.

Gute Chlorwerte sind durch Kombination mehrerer Reinigungsschritte zu erzielen: Eine Kombination von Filtration und Umkristallisation zur Reinigung von Biphephos zeigt WO 2012/095255A1. Dabei wird ein festes Organophosphorprodukt in einem Lösungsmittel aufgeschlämmt und mittels einer Fritte filtriert. Das dabei erhaltene, wiederum feste Organophosphorprodukt enthielt neben dem gewünschten Biphephos 2500 ppm Gesamtchlor. Um den Gesamtchlorgehalt zu reduzieren, wurde der Feststoff in einem Lösungsmittel suspendiert, erhitzt und gefiltert. Sodann wurde das Filtrat umkristallisiert. Das dabei erhaltene, gereinigte Organophosphorprodukt wies einen Gesamtchlorgehalt von lediglich 35 ppm auf.

Zwar ist ein solcher Chlorgehalt durchaus befriedigend, jedoch erfordert dieses Reinigungsverfahren viele Arbeitsschritte und für die Umkristallisation große Mengen verschiedener Lösungsmittel, sodass es sich eher für den Labormaßstab eignet als für die industrielle Ligandensynthese.

Ausgehend von diesem Stand der Technik liegt der Erfindung die Aufgabe zu Grunde, eine industriell praktikable Aufreinigungsmethode anzugeben, bei der der Chlorgehalt von einem Organophosphorprodukt, welches mindestens eine Organophosphorverbindung, sowie mindestens eine Chlorverbindung als Verunreinigung enthält, von ursprünglich 1000 bis 100.000 ppm auf einem Gesamtchlorgehalt zwischen 10 ppm und 10.000 ppm reduziert werden kann. Die angestrebten Chlorgehalte verstehen sich dabei als Gesamtchlorgehalt, also sowohl auf organisch gebundenes Chlor als auch anorganisch gebundenes Chlor. Die Einheit ppm steht für 10⁻⁶ und bezieht sich auf die jeweiligen Gewichte.

Des Weiteren soll das Reinigungsverfahren aufgrund der Hydrolyseinstabilität der Organophosphite auf die Verwendung von Wasser verzichten. Schließlich soll aus Umweltschutzgründen von der Verwendung von großen Lösungsmittelmengen - wie sie bei der Extraktion oder Umkristallisation notwendig sind - abgesehen werden.

Des Weiteren wäre es wünschenswert, ein Verfahren anzugeben, welches universell für unterschiedliche Ligandenklassen eingesetzt werden kann. Dies führt zu einer besseren Automatisierbarkeit der Ligandensynthese und damit zu einer höheren Produktqualität. Dies ist insbesondere für großtechnische Synthesen und Verfahrensentwicklungen vorteilhaft, da das gleiche Equipment verwendet werden kann und auf teure Investitionen von neuen Anlagen verzichtet werden kann. Auch sollte die Anzahl der notwendigen Arbeitsschritte möglichst gering sein, um die Fehleranfälligkeit und die Produktionszeit zu verringern.

Gelöst werden diese Aufgaben durch ein Verfahren der eingangs genannten Gattung mit den Schritten:
a) Bereitstellen des verunreinigten Organophosphorprodukts;
b) vollständiges Auflösen des verunreinigten Organophosphorprodukts in einem Lösungsmittel unter Erhalt einer verunreinigten Lösung;
c) Trennung der verunreinigten Lösung mittels einer Membrantrenneinheit unter Erhalt einer gereinigten Lösung;
d) Entfernung des Lösungsmittels aus der gereinigten Lösung unter Erhalt eines gereinigten Organophosphorprodukts.

Gegenstand der Erfindung ist mithin ein Verfahren zur Reinigung eines verunreinigten Organophosphorprodukts, welches mindestens eine Organophosphorverbindung, sowie als Verunreinigung mindestens eine Chlorverbindung enthält mit den Schritten a) bis d).

Die erfindungsgemäße Reinigung findet mittels einer Membran statt. Dabei können auch mehrere Membrantrennschritte und Filtrationsschritte kombiniert werden. Darüber hinaus können sowohl Filtrationsschritte, als auch Membrantrennschritte ausgeführt werden.

Das Reinigungsverfahren kommt ohne Wasser und ohne unbotmäßigen Verbrauch von Lösungsmitteln aus. Dennoch stellt das Lösungsmittel ein wichtiges Element der Erfindung dar, da die verunreinigte Organophosphorverbindung vor der Reinigung mittels Filter bzw. Membrantrenneinheit vollständig in dem Lösungsmittel aufgelöst werden muss.

Das vollständige Auflösen des Organophosphorprodukts stellt ein wesentliches Unterscheidungsmerkmal der vorliegenden Erfindung gegenüber anderen Filtrationsverfahren, wie beispielsweise die aus EP2091958B1 oder WO 2012/095255A1 bekannte Verfahren dar:
Unter einem "vollständigen Auflösen" des verunreinigten Organophosphorprodukts ist zu verstehen, dass sowohl die gewünschte Organophosphorverbindung, als auch die unerwünschten Chlorverbindungen vollumfänglich in dem Lösungsmittel aufgelöst werden. Es darf mithin kein Feststoff mehr in der Lösung erkennbar sein. Die Sichtprüfung genügt für den erfindungsgemäß angestrebten Erfolg. Freilich werden sich in einer optisch vollständigen Lösung mit geeigneten, objektiven Messmethoden noch Restpartikel nachweisen lassen. Jedoch soll das erfindungsgemäße Verfahren auch einfach zu praktizieren sein. Deshalb wird für die Beurteilung einer vollständigen Lösung im Sinne der Erfindung eine Sichtprüfung als hinreichendes Kriterium angesehen.

Die im Stand der Technik bekannten, lösungsmittelbasierten Filtrationsverfahren arbeiten dagegen nicht mit einer vollständigen Auflösung des verunreinigten Organophosphorprodukts; vielmehr ist es in der Ligandensynthese üblich, die Organophosphorverbindung als Suspension oder Slurry in dem Lösungsmittel bereitzustellen und dann zu filtrieren. So genügt die bei der Synthese der Struktur D-1 verwendete Menge Toluol in EP2091958B1 nicht, um die Substanz D-1 vollständig darin aufzulösen. In WO 2012/095255A1 ist explizit von Suspendieren oder Aufschlämmen die Rede, aber nicht von einer vollständigen, feststofffreien Lösung.

Das vollständige Auflösen des verunreinigten Organophosphorprodukts einschließlich der Chlorverschmutzung in dem Lösungsmittel erlaubt eine selektivere Trennung der Organophosphorverbindung von der Chlorverbindung an dem Filter oder an der Membran. Bei dem Trennungsschritt c) fällt eine gereinigte Lösung an, aus der die Chlorverbindung abgereichert ist. Nach Entfernen des Lösungsmittels aus der gereinigten Lösung - dies geschieht in an sich bekannter Weise wie z. B. durch Destillation - wird ein gereinigtes Organophosphorprodukt erhalten, dessen Gesamtchlorgehalt deutlich geringer ist als der des ursprünglich bereitgestellten, verunreinigten Organophosphorprodukts. Das entfernte Lösungsmittel kann rezykliert und erneut zum Auflösen von verunreinigtem Organophosphorprodukt verwendet werden. Der Entsorgungsaufwand für das Lösungsmittel sinkt dadurch.

In einer Ausführungsvariante der Erfindung werden sowohl Filtrationsschritte, als auch Membrantrennschritte ausgeführt.

Das erfindungsgemäße Verfahren macht es möglich, den Gesamtchlorgehalt eines verunreinigten Organophosphorprodukts, welcher zwischen 1.000 und 100.000 ppm liegt, im gereinigten Zustand auf einen Wert zwischen 10 und 10.000 ppm zu reduzieren. Vorzugsweise führt das Reinigungsverfahren zu einem Gesamtchlorgehalt des gereinigten Organophosphorprodukts zwischen 10 und 1.000 ppm. Die Einheit ppm steht dabei für millionstel Gewichtsanteil. Der Gesamtchlorgehalt umfasst sowohl organische, als auch anorganische Spezies. Ein geeignetes Verfahren zur Bestimmung des Gesamtchlorgehalts ist die Verbrennung nach Wickbold mit Probenvorbereitung nach DIN 51408 und Messung per lonenchromatographie nach DIN EN ISO 10304.

Das verunreinigte Organophosphorprodukt kann nämlich organische Chloride und/oder anorganische Chloride enthalten. Organische Chloride enthalten mindestens ein Kohlenstoffatom, währenddessen anorganische Chloride kein Kohlenstoff aufweisen. Verschmutzungen des Organophosphorprodukts durch die folgenden Chloride sind besonders wahrscheinlich, da diese chlorhaltigen Verbindungen entweder bei der Synthese von Organophosphorverbindungen benötigt werden oder als Nebenprodukte unvermeidlich anfallen:
Phosphortrichlorid, Chlorophosphite, Dichlorophosphite, Hydrochloride von Aminen, Hydrochloride von Alkalimetallen, Chloride von Erdalkalimetallen, chlorhaltige Säuren erhältlich aus der Hydrolyse von Phosphortrichlorid.

Deshalb weist das verunreinigte Organophosphorprodukt bevorzugt mindestens eines der aufgezählten Chloride auf.

Als Lösungsmittel wird eine Substanz gewählt, welche sowohl die Organophosphorverbindung, als auch die chlorhaltige Verunreinigung zu lösen vermag. Andernfalls wäre eine vollständige Auflösung des verunreinigten Organophosphorprodukts darin nicht möglich. Als Lösungsmittel kann entweder eine Reinsubstanz der folgenden Auflistung verwendet werden oder aber ein Gemisch mehrerer Substanzen dieser Gruppe:
- aromatische Kohlenwasserstoffe wie insbesondere Toluol, Xylol, o-Xylol, Kresol;
- Ether, wie insbesondere Tetrahydrofuran (THF), Petrolether, Diethylether, Methyl-tert-butylether (MTBE);
- Nitrile wie insbesondere Acetonitril (ACN);
- Ethylacetat;
- Aceton;
- Alkohole wie insbesondere Methanol, Ethanol, Isopropanol, Butanol.

Bei den eben genannten Lösungsmitteln handelt es sich um organische Lösungsmittel. Die verunreinigte Lösung, also das in dem organischen Lösungsmittel vollständig aufgelöste, verunreinigte Organophosphorprodukt, bildet mithin eine organische Phase. Da das Verfahren wegen der Hydrolyseanfälligkeit der Organophosphorprodukte vorzugsweise in Abwesenheit von Wasser durchgeführt wird, entsteht neben der organischen Phase keine wässrige Phase. Aufgrund der vollständigen Auflösung ist die verunreinigte Lösung feststofffrei. Die von der verunreinigten Lösung gebildete organische Phase ist somit die einzige Phase in dem aus Organophosphorprodukt, Verunreinigung und Lösungsmittel gebildeten System.

In Abhängigkeit von dem Lösungsmittel und insbesondere der Organophosphorverbindung ist eine vollständige Auflösung des verunreinigten Organophosphorprodukts bei Raumtemperatur nicht immer möglich. Deswegen sieht eine bevorzugte Weiterbildung der Erfindung es vor, das verunreinigte Organophosphorprodukt bei erhöhter Temperatur aufzulösen. Die Temperatur sollte dabei so gewählt werden, dass das Lösungsmittel noch nicht zu sieden beginnt. Die Temperatur des eingesetzten Lösungsmittels sollte deswegen zwischen 20 °C und der Siedetemperatur des gewählten Lösungsmittels liegen. Optimal ist ein Temperaturbereich von 40 °C bis 120 °C. Die Erhöhung der Temperatur des Lösungsmittels ermöglicht es auch, die Lösungsmittelmenge zu reduzieren. Dies ist insbesondere dann von Vorteil, wenn ein gesundheitlich bedenkliches, umweltgefährdendes oder schlichtweg teures Lösungsmittel eingesetzt wird.

In einer Ausführungsvariante der Erfindung werden sowohl Filtrationsschritte, als auch Membrantrennschritte ausgeführt.

Das Grundwissen des Fachmanns auf dem Gebiet der Filtration ist durch die folgende Literatur belegt:
Pongratz et al., Handbuch der industriellen Fest-/Flüssigfiltration (2000);
Sparks, Trevor: Solid-Liquid Filtration - A Users' Guide to Minimizing Costs and Environmental Impact; Maximizing Quality and Productivity. Elsevier (2012);
Cheremisinoff, Nicholas P.: Liquid Filtration (2nd Edition). Elsevier (1998)
Sutherland: Filters and Filtration Handbook (2008).

Eine besonders bevorzugte Ausführungsform der Membrans- und Filtrations-Variante sieht vor, dass die Trennung der verunreinigten Lösung in Gegenwart eines Filtrationshilfsmittels durchgeführt wird. Das Filtrationshilfsmittel wird dafür entweder unmittelbar vor der Filtration der verunreinigten Lösung oder noch früher dem Lösungsmittel zugegeben. Filtrationshilfsmittel binden die abzutrennenden Verunreinigungen zum Teil physikalisch und verhindern dadurch ein Überwinden des Filters. In der Regel bilden die abzutrennenden Partikel eine Schicht auf der Filteroberfläche und bilden Brücken über den Poren des Filters. Erst diese Brücken bewirken meist eine vollständige Abtrennung der Partikel. Zu Beginn gibt es daher i.d.R. noch einen Durchbruch der Partikel. Die Anfiltration mit Filterhilfsmitteln hat hier den Vorteil diese Brücken bereits mit dem Filterhilfsmittel aufzubauen und einen Durchbruch der Partikel aus dem Produktgemisch zu verhindern.

Als Filtrationshilfsmittel können sowohl mineralische Filtrationshilfsmittel wie beispielsweise Siliziumdioxid oder organische Filtrationshilfsmittel wie beispielsweise Cellulose oder Aktivkohle verwendet werden. Es können auch unterschiedliche Filtrationshilfsmittel gemischt werden.

Im Gegensatz zu einer Filtrierung basiert die Membrantrennung nicht nur auf dem Siebeffekt, sondern darüber hinaus auch auf Lösungs- und Diffusionseffekten. Ein weiterer Unterschied gegenüber der Filtration besteht darin, dass die mittels Membrantechnik abzutrennenden Stoffe durchaus die Membran überwinden können; dies jedoch nur mit verringerter Geschwindigkeit. Die selektive Trennung erfordert in der Praxis meist zusätzlich die Einstellung entsprechender Überstromgeschwindigkeiten. Auch setzt die Membrantrennung ein deutliches Druckgefälle zwischen beiden Seiten der Membran voraus, den sogenannten Transmembrandruck, wohingegen bei der Filtration der Druckverlust deutlich geringer ist. Ein Transmembrandruck ist jedoch nicht immer zwingend erforderlich, zum Beispiel dann, wenn die treibende Kraft aus einem unausgeglichenen Lösungsgleichgewicht resultiert (Umkehrosmose). Eine Membrantrennung ist mithin deutlich komplexer und technisch anspruchsvoller als eine Filtration. Gleichwohl ermöglicht sie die Abtrennung von Verunreinigungen, die sich im Wege einer klassischen Filtration nicht entfernen lassen.

Eine Einführung in die Membrantechnologie bieten Melin / Rautenbach: Membranverfahren. Grundlagen der Modul- und Anlagenauslegung. Springer, Berlin Heidelberg 2004.

Es hat sich gezeigt, dass Chlorverbindungen besonders gut die Membran permeieren. Aus diesem Grunde fällt bei der Membrantrennung der verunreinigten Lösung die gereinigte Lösung vor der

Membran, also als Retentat an. Auch hier zeigt sich ein wesentlicher Unterschied gegenüber einer Filtration, wo die Verunreinigung vom Filter zurückgehalten wird. Als Retentat wird in der Membrantechnik der Stoffstrom von einer Membrantrenneinheit bezeichnet, der aus Sicht des anströmenden Zulaufs vor der Membran abgezogen wird. Der Stoffstrom, der hinter der Membran abgezogen wird, wird als Permeat bezeichnet, da er die Membran überwunden hat. Zu den Begrifflichkeiten der Membrantrenntechnik wird auf Melin/Rautenbach verwiesen.

Ein wichtiges Erfolgskriterium bei der Anwendung der Membrantechnik ist die Auswahl des zur Trennaufgabe passenden Membranmaterials. Aufgrund der komplexen Wechselwirkung zwischen Membran und den zu trennenden Stoffen ist die Identifikation des Membranmaterials alles andere als trivial. Hinzu kommt, dass die Membran gegenüber dem eingesetzten Lösungsmittel stabil sein muss. Zur Aufreinigung des chlorverschmutzten Organosphosphorprodukts haben sich silikonbasierte Polymere oder Polyethersulfon als trennaktive Membranmaterialien besonders hervorgetan, da sie für die typischen Chloride eine deutlich höhere Durchlässigkeit aufweisen als für Organophosphorverbindungen. Polyimid ist ebenfalls geeignet.

Die Membrantrenneinheit kann eine Mehrzahl von Membranmodulen enthalten, die unterschiedlich miteinander verschaltet sein können. Es kommen dabei die üblichen Modulverschaltungen in Betracht, wie sie beispielsweise Melin/Rautenbach schildern. Die Membranmodule selbst können konstruktiv in unterschiedlichen Varianten ausgeführt sein, zum Beispiel als Taschenmodule, Plattenmodule, Wickelmodule, Hohlfasermodule, Kapillarmodule oder Schlauchmodule.

Neben der Bauart haben auch Prozessparameter der Membrantrennung Einfluss auf das Trennergebnis. Wichtige Betriebsparameter sind das ausgewählte Lösungsmittel, der Transmembrandruck und die Überstromgeschwindigkeit. Der Membrantechniker weiß diese Betriebsparameter geeignet zu wählen.

Eine besondere Bedeutung hat die Trenntemperatur, also die Temperatur, mit der die verunreinigte Lösung auf die Membran gegeben wird. Die Permeabilität vieler Membranmaterialien ist nämlich abhängig von der Temperatur. Da der Temperatur des Lösungsmittels beim Auflösen des verunreinigten Organophosphorprodukts ebenso eine große Bedeutung zukommt, sieht eine bevorzugte Weiterbildung der Erfindung es vor, dass die Membrantrennung bei einer Temperatur erfolgt, die von der Temperatur beim vollständigen Auflösen des verunreinigten Organophosphorprodukts mit Lösungsmittel abweicht. Demnach findet zwischen Schritt b) und Schritt c) entweder eine bewusste Erwärmung oder bewusste Abkühlung des verunreinigten Lösungsmittels statt. Ob konkret gekühlt oder geheizt werden muss, hängt von dem verwendeten Lösungsmittel, den darin gelösten Organophosphorverbindungen und Chloriden, sowie dem trennaktiven Membranmaterial ab. In der Regel wird die Trennung der verunreinigten Lösung mittels der Membrantrenneinheit bei einer Temperatur von 10 bis 80°C erfolgen, besonders bevorzugt 20 bis 60°C.

Wie eingangs dargelegt, existiert eine Vielzahl von technisch relevanten Organophosphorverbindungen, die zu den unterschiedlichsten Zwecken verwendet werden. Das Primat der Chlorfreiheit kann deswegen bei unterschiedlichen Einsatzzwecken der unterschiedlichen Organophosphorverbindungen unterschiedlich stark ausgeprägt sein. Besonders hohe Anforderungen an den Gesamtchlorgehalt von Organophosphorprodukten wird dort gestellt, wo die eigentliche Organophosphorverbindung als Ligand in einer homogen katalysierten, kontinuierlich betriebenen Reaktion verwendet wird, deren Reaktionsapparaturen durch Chloride geschädigt werden.

Organophosphorverbindungen, die typischerweise als Liganden in homogenen Katalysatorsystemen eingesetzt werden, sind Phosphine, Diphosphine, Phosphonite, Phosphinite, Phosphoramidite, Monophosphite und Bisphosphite. Das erfindungsgemäße Reinigungsverfahren findet somit insbesondere im Zusammenhang mit der Darstellung dieser Substanzklassen Anwendung.

Besonders bevorzugt dient es zur Aufreinigung von Organophosphorprodukten, die Phosphoramidite oder Phosphite enthalten. Es ist insbesondere dafür bestimmt, Bisphosphite aufzureinigen, wobei es sich zur Aufreinigung der Bisphosphite der Strukturformeln (I) bis (IV) besonders eignet:

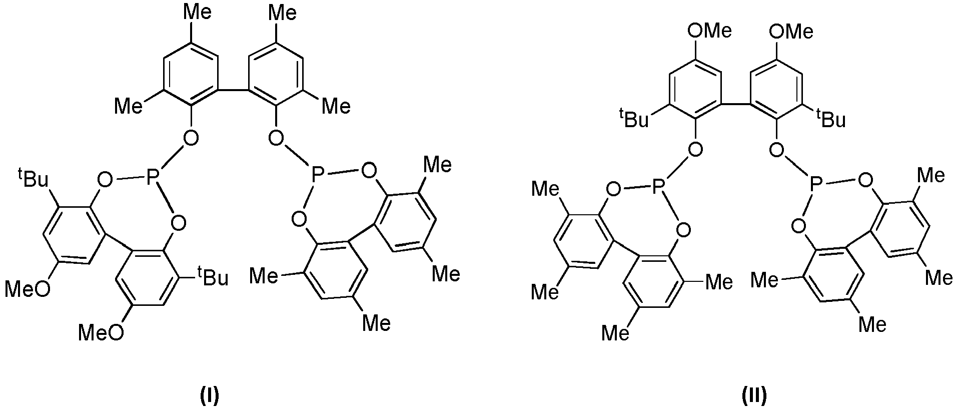

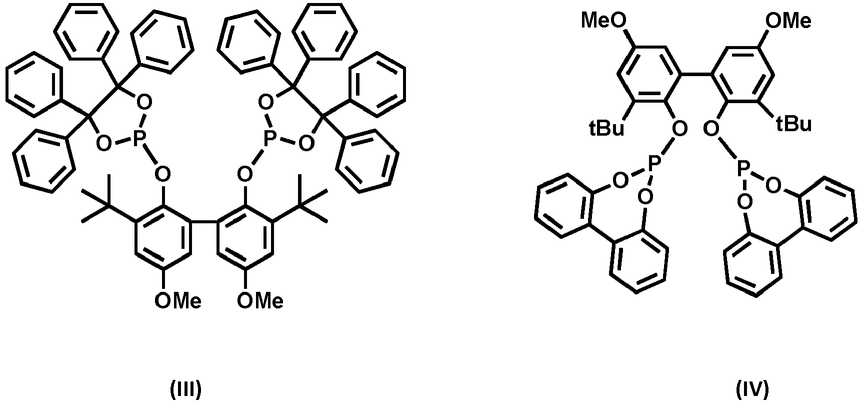

Aufgrund der besonderen Eignung des beschriebenen Verfahrens zur Reinigung von bei der Ligandensynthese anfallenden Organophosphorprodukten, ist ein Verfahren zur Herstellung eines Katalysatorsystems enthaltend ein Metall sowie mindestens eine Organophosphorverbindung als Ligand ebenfalls Gegenstand der Erfindung, sofern bei der Herstellung des Katalysatorsystems ein erfindungsgemäß gereinigtes Organophosphorprodukt verwendet wird, welches solche Organophosphorverbindungen enthält, die typischerweise als Ligand in Katalysatorsystemen verwendet werden.

Die Herstellung des Katalysatorsystems erfolgt meist nicht an dem Ort, an dem auch die Ligandensynthese erfolgt, sondern in situ, also dort, wo das Katalysatorsystem eingesetzt wird, nämlich im Reaktor. Die Herstellung des Katalysatorsystems erfolgt dabei dadurch, dass das ex situ hergestellte und erfindungsgemäß gereinigte Organophosphorprodukt zusammen mit dem Metall in den Reaktor eingefüllt wird. Das eigentliche Katalysatorsystem formiert sich erst im Reaktor in Gegenwart der Reaktionsteilnehmer.

Da die Dank des erfindungsgemäßen Verfahrens erzielte hohe Reinheit des Organophosphorprodukts seine Vorteile letztendlich erst am Einsatzort des Organophosphorprodukts entfaltet, ist die Durchführung von Reaktionen in Reaktoren, die besonders empfindlich auf die entfernten Chloride reagieren, ebenfalls erfindungsgegenständlich und zwar dann, wenn ungesättigte Verbindungen in Gegenwart eines homogenen Katalysatorsystems hydroformyliert oder hydrocyaniert oder hydriert werden, sofern das Katalysatorsystem ein Metall sowie mindestens eine Organophosphorverbindung als Ligand aufweist, und sofern bei der Herstellung des Katalysatorsystems das entsprechende Organophosphorprodukt erfindungsgemäß gereinigt wurde. Besonders gefährdet sind Reaktoren aus Stahl, sodass das Reinigungsverfahren insbesondere dann vorteilhaft eingesetzt wird, wenn die katalysierte Reaktion in einer stählernen Apparatur erfolgt.

### Beispiele

Die erfindungsgemäße Reinigung chlorverschmutzter Organophosphorverbindungen soll nun anhand von einigen Ausführungsbeispielen erläutert werden. Dabei werden die folgenden Abkürzungen verwendet:
ACN = Acetonitril
KPG = Kerngezogenes Präzisions-Glasgerät
VE-Wasser = demineralisiertes Wasser
EtOAc = Ethylacetat
acac = acetylacetonat
NEt₃ = Triethylamin
DMAB = Dimethylaminobutan
KO^{t}Bu = Kalium-tert-butylat
RT = Raumtemperatur

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Die Chlorbestimmung erfolgte als Verbrennung nach Wickbold; mit Probenvorbereitung nach DIN 51408 und Messung per lonenchromatographie nach DIN EN ISO 10304.

### Herstellung des Liganden (III):

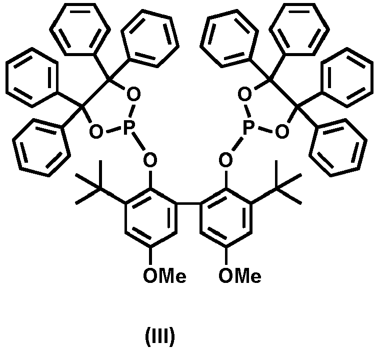

Die Synthese des Bisphosphitliganden (III) erfolgte analog den Vorschriften in DE 102006058682. Als Base wurde jedoch KOtBu (Kalium-tert-butylat) verwendet.

In einem 2000 mL Schlenkkolben wurde 53.0 g (0,122 mol) 1-Chlor-3,3,4,4-tetraphenylphospholan (Chlorophosphit) vorgelegt und in 800 ml getrocknetem Acetonitril gelöst und zwei Stunden gerührt. In einem zweiten sekurierten Schlenkkolben (1000 ml) wurden 15,8 g (0,043 mol) 3,3'-Di-tert.-butyl-5,5'-dimethoxy-[1,1'-biphenyl]-2,2'diol und 13,9 g (0,123 mol) Kalium-tert.-butylat abgewogen und 800 ml getrocknets Acetonitril zugegeben. Anschließend wurde diese Aufschlämmung bei RT unter Rühren über einen Zeitraum von 3,5 h in die "Chlorophosphit" - Lösung getropft und über Nacht bei RT gerührt.

Zur Aufarbeitung wurde der erhaltene Feststoff filtriert und viermal mit 200 ml entgastem ACN gewaschen. Das restliche Lösungsmittel wurde bei 10⁻¹ mbar für 3 h bei 40 °C unter vermindertem Druck entfernt und es wurden 43,9g (88 %) des Rohliganden (III) erhalten.
³¹P{¹H}-NMR (C₆D₆, 202 MHz): δ = 145,62 ppm. ¹H-NMR (500 MHz, C₆D₆): δ = 1,39 (s, 18 H), 3,32 (s, 6 H), 6,84-6,88 (m, 7 H), 6,89-6,94 (m, 7 H), 6,95-7,00 (m, 4 H), 7,04-7,09 (m, 8 H), 7,21-7.30 (m, 10 H), 7.57 (d, J=7,5 Hz, 4 H), 7,65 (d, J=7,5 Hz, 4 H) ppm. Elementaranalyse (ber. für C₇₄H₆₈O₈P₂ = 1147,29 g/mol): C 77,00 (77,47); H 5,66 (5,97); P 5,82 (5,40) %.

### Reinigung des Liganden (III):

27,1g des Rohliganden (III) wurden in 1900 ml entgastem Toluol über Nacht bei RT gelöst (Cl-Wert nach Wickbold des Rohliganden (III): 7,5 Gew %). Der Ansatz wird in ein mit Stickstoff gespültes 2 Liter Druckfiltrationsgerät mit einer Filterfläche von 130 cm² überführt und durch Ultrafiltrationsmembran der Firma Microdyn Nadir vom Typ UV150T getrennt. Die Ultrafiltration gehört zu den Membranverfahren, wenngleich der Name anderes vermuten lässt. Die Trenntemperatur lag bei 20°C und der Transmembrandruck bei 0,4 bar. Der mittlere Permeatfluss während der gesamten Membrantrennung lag bei 57 ml/min.

Das erzeugte Retentat hatte einen Chlorwert, ermittelt nach Wickbold, von 55 mg/kg.

Der Chlorwert konnte somit bei einer Ligandausbeute von 81,2% um 99,9% reduziert werden.

### Herstellung des Liganden (I):

Die Organophosphorverbindung 4,8-di-tert-butyl-2,10-dimethoxy-6-((3,3',5,5'-tetramethyl-2'-((2,4,8,10-tetramerthyldibenzo[d,f][1,3,2]dioxaphosphepin-6-yl)oxy)-[1,1'-biphenyl]-2-yl)oxy)dibenzo[d,f][1,3,2]dioxaphosphepin, mit der Strukturformel (I) 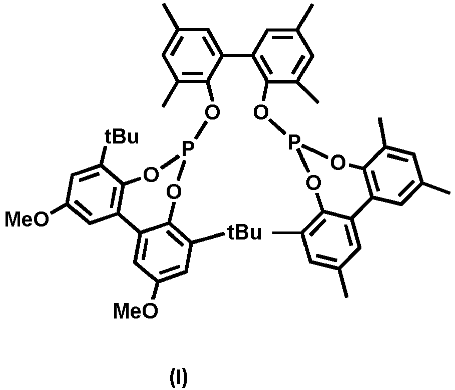 wird hier als Ligand (I) bezeichnet. Die Synthese dieses Liganden erfolgte nach den Vorschriften der zum Zeitpunkt der Anmeldung noch unveröffentlichten deutschen Patentanmeldungen 102013219506.0 und 102013219508.7:

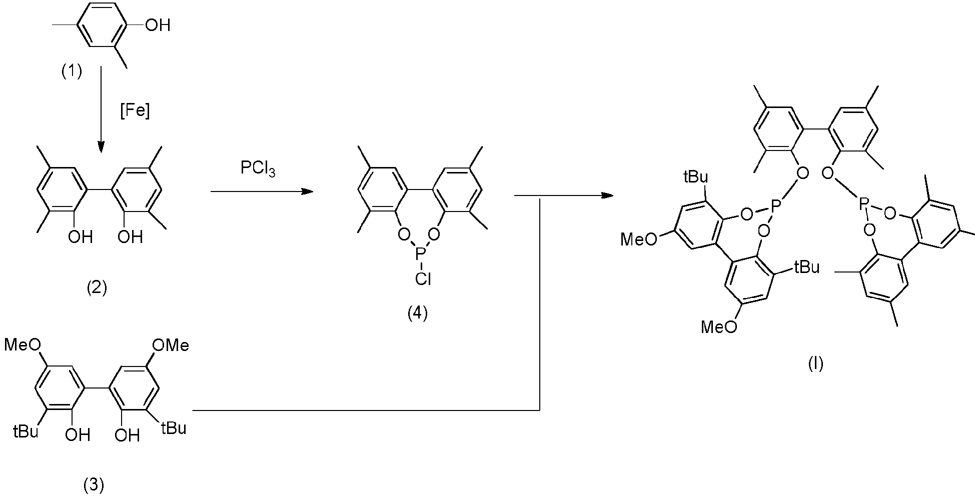

### Synthese des 2,2'-Bis(3,5-dimethylphenol)s (2):

Das als Vorstufe eingesetzte Biphenol (2) wurde nach folgender Synthesevorschrift hergestellt.

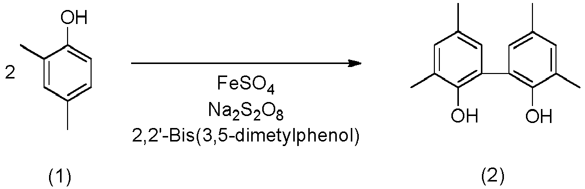

In einem 500 ml Schlenk mit KPG-Rührer, Zwischenaufsatz und Glasrührer wurden 1,42 g (0,005 mol) Eisen(II)-sulfatheptahydrat und 12,35 g (0,1 mol) 2,4-Dimethylphenol in 150 ml VE-Wasser und 5 ml Cyclohexan vorgelegt und auf 40 °C erwärmt.

In einem 100 ml Becherglas löste man 25.36 g (0,146 mol) Natriumperoxodisulfat in 80 ml VE-Wasser. Zum Start der Reaktion wurde eine kleine Portion Na₂S₂O₈-Lösung zum Phenol gegeben. Anschließend wurde alle 10 min eine kleinere Portion der Lösung hinzugegeben. Nach 30 min war die Na₂S₂O₈-Lösung Zugabe beendet.

Nach einer Reaktionszeit von 5h wurde zur Reaktionslösung 300 ml Cyclohexan und 200 ml Wasser hinzugegeben, 20 min rühren gelassen, dann warm in den Scheidetrichter überführt.

Die organische Phase wurde abgetrennt und bis zur Trockene eingeengt. Das Produkt (5) konnte in 69%iger Ausbeute (10,6 g) erhalten werden.

### Synthese des 2,2'-Bis-(3,5-dimethylphenol)chlorophosphits (4):

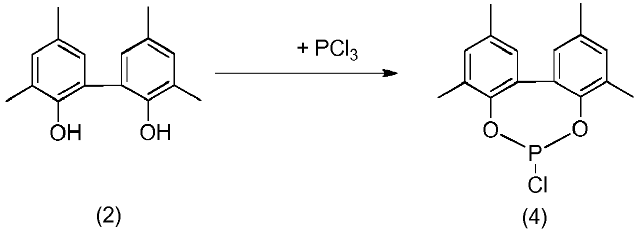

In einem sekurierten 2 L Schlenk mit Magnetrührer wurden 440 ml Phosphortrichlorid vorgelegt. In einem zweiten sekurierten 1 L Schlenk wurden 120 g 2,2' -Bis-(3,5-dimethylphenol) eingewogen und unter Rühren 500 ml getrocknetes Toluol hinzugefügt. Die Biphenol-Toluol-Suspension wurde innerhalb von 4 h bei 63°C zum Phosphortrichlorid dosiert. Nach vollständiger Zugabe wurde die Reaktionsmischung über Nacht bei Temperatur gerührt. Am nächsten Morgen wurde die Lösung in der Wärme (45°C) eingeengt und das Produkt konnten in 96,5%iger Ausbeute (153 g) erhalten werden. ³¹P-NMR: 175,59 (94,8% 2,2'-Bis-(3,5-dimethylphenol)chlorophosphit), 4,4% diverse PCI-Verbindungen, 0,8% P-H-Verbindung.

### Allgemeine Vorschrift zur Herstellung des Liganden (I):

### Variante 1, in ACN/NEt₃:

In einem 1000 ml Schlenkkolben wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenkkolben (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem Triethylamin (0,29 mol) versetzt. Dann wurde langsam die Biphenol/Trietylamin-Lösung zu der Chlorophosphitlösung getropft. Nach einer Nachreaktionszeit von 1h wurde die Reaktionslösung über Nacht bei 45°C gerührt.

Anschließend wurde die Lösung filtriert und der Feststoff dreimal mit 100 ml warmen (45°C) ACN gewaschen. Das Rohprodukt konnte als weißer Feststoff (43,3g, 86%) erhalten werden. ³¹P-NMR (202,4 MHz, toluene-d₈): 142,5 und 140,9 ppm. Der Chlorgehalt sollte später zu 3,5 Gew.-% bestimmt werden.

### Variante 2, in ACN/DMAB:

In einem 100 ml Schlenkkolben wurde unter Schutzgas 6 g (19,0 mmol) 2,2'-Bis-(3,5-dimethylphenyl)chlorophosphit in 20 ml entgastem ACN gelöst und auf 35°C erwärmt. In einem zweiten Schlenkkolben (50 ml) wurden 3,4 g (9,0 mmol) 3,3'-Di-tert.-butyl-5,5'-dimethoxy-[1,1'-biphenyl]-2,2'diol in 15 ml Dimethylaminobutan (DMAB) gelöst und anschließend langsam zu der Chlorophosphitlösung getropft. Die Reaktion wurde bei 35°C über Nacht rühren gelassen.

Am nächsten Tag wurde die Lösung filtriert und der Feststoff zweimal mit ACN gewaschen. Das Rohprodukt konnte als weißer Feststoff (5,3g, 66%) erhalten werden. ³¹P-NMR (202,4 MHz, toluene-d₈): 142,8 und 141,2 ppm

### Variante 3, in EtOAc/NEt₃:

In einem 100 ml Schlenkkolben wurde unter Schutzgas 7,3 g (21,0 mmol) 2,2'-Bis-(3,5-dimethylphenyl)chlorophosphit in 15 ml entgastem Ethylacetat gelöst und auf 35°C erwärmt. In einem zweiten Schlenkkolben (100 ml) wurden 3,9 g (9,5 mmol) 3,3'-Di-tert.-butyl-5,5'-dimethoxy-[1,1'-biphenyl]-2,2'diol in 7,0 ml NEt₃ gelöst. Anschließend wurde die Biphenol/Triethylamin-Lösung langsam innerhalb von 20 Minuten zu der Chlorophosphitlösung getropft. Die Lösung wurde eine weitere Stunde bei 35°C und anschließend über Nacht bei 45°C gerührt.

Dieser Feststoff wurde in entgastem ACN 1.5h bei 75°C suspendiert und abgetrennt und mit warmen ACN nachgewaschen. Anschließend wurde das Produkt in getr. Toluol 1.5h bei 35°C suspendiert und abgetrennt.

Das Rohprodukt konnte als weißer Feststoff (5.0 g, 58%) erhalten werden. ³¹P-NMR (202,4 MHz, toluene-d₈): 142,5 und 140,9 ppm. Der Chlorgehalt sollte später zu 4,5 Gew.-% bestimmt werden.

### Aufreinigung des Liganden (I) mit Hilfe von Filtration und Membrantrennung:

95 g des Rohliganden (I), hergestellt nach Variante 1, wurde in 1900 ml Toluol bei 75°C innerhalb von 45 Minuten gelöst (Cl-Wert nach Wickbold des Rohliganden (I): 3,5 Gew %) und in ein mit Stickstoff gespültes 2 Liter Druckfiltrationsgerät mit einer Filterfläche von 130 cm² überführt und durch das Filtertuch Propex 14K filtriert. Die Filtrationstemperatur lag bei 20°C und der Filtrationsdruck bei 0,5 bar. Die Filtrationszeit betrug 9 Minuten.

Das Filtrat wurde wiederum unter inerten Bedingungen durch eine Filterschicht der Firma PALL vom Typ Seitz ® K 100 mit einer Fläche von 20 cm² filtriert. Eine Probe des erzeugten Filtrats ergab folgenden Chlorwert, ermittelt nach Wickbold: 820 mg/kg.

Das restliche Filtrat von 1550 g wurde abschließend in einer gerührten Dead-End Trennzelle der Firma Evonik Membrane Extraction Technology Limited vom Typ METCELL® mit einer Nanofiltrationsmembran getrennt. Verwendet wurde eine organophile Nanofiltrationsmembran der Firma GMT Membrantechnik GmbH vom Typ ONF-2. Die Membranfläche beträgt 54 cm². Die aktive Membranfläche ist dem Feed zugewandt verbaut. Das Permeat fließt durch die Sintermetalplatte der Trennzelle über den Permeatauslass ab. Die Membrantrennung wurde bei 27°C und einem Stickstoffdruck von 30 bar durchgeführt. Es wurden 1137 g Permeat erzeugt. Dies entspricht einem volumetrischen Konzentrierungsfaktor von 3,8.

Das erzeugte Permeat enthält 8 g Feststoff sowie einen Chlorwert nach Wickbold von 880 mg/kg und enthielt vor allem die chlorhaltigen verunreinigungen als Hauptbestandteil.

Das erzeugte Retentat enthält 38 g Feststoff sowie einen Chlorwert nach Wickbold von 260 mg/kg.

Der Chlorwert konnte somit um 98% reduziert werden.

### Aufreinigung des Liganden (I), hergestellt nach Variante 3 mittels Membrantechnik und Filtration mit SiO₂-Filterhilfsmittel:

92,1 g des Rohliganden (I), hergestellt nach Variante 3, wurden in 1900 ml Toluol bei 75°C gelöst (Cl-Wert nach Wickbold des Rohliganden (I): 4,5 Gew %). Das größtenteils auf SiO₂ basierende Filterhilfsmittel Hyflo SuperCel wurde bei 80°C für 14 Stunden in einem Trockenschrank getrocknet. 20 g getrocknetes Hyflo SuperCel wurden in 400 ml getrocknetem Toluol für 2 Stunden eingerührt und danach in ein mit Stickstoff gespültes 2 Liter Druckfiltrationsgerät mit einer Filterfläche von 130 cm² überführt und auf einer Ultrafiltrationsmembran anfiltriert. Als Ultrafiltrationsmembran wurde eine Membran der Firma Microdyn Nadir vom Typ UV150T verwendet. Die Trenntemperatur lag bei 20°C und der Transmembrandruck bei 0,4 bar. Anschließend wurde die Rohlösung langsam und stickstoffüberlagert in das Druckfiltergerät überführt und danach bei 20°C und 0,4 bar filtriert.

Der Cl-Wert des erzeugten Filtrats betrug 190 mg/kg.

Der Chlorwert konnte somit bei einer Ligandenausbeute von 77,6% um mehr als 99% reduziert werden.

### Aufreinigung des Liganden (I), hergestellt nach Variante 2 mittels Filtration und Cellulose-Filterhilfsmittel (Vergleichsversuch):

73,2 g des Rohliganden (I), hergestellt nach Variante 2, wurden in 1500 ml Toluol bei 75°C über Nacht gelöst (Cl-Wert Nach Wickbold des Rohliganden: 1,0 Gew %) und mit 10 g des auf Cellulose basierenden Filterhilfsmittels Vitacell LC200 versetzt.

Diese Rohlösung enthält bereits das aus Cellulose bestehende Filterhilfsmittel VITACEL ® LC 200 und in ein mit Stickstoff gespültes 2 Liter Druckfiltrationsgerät mit einer Filterfläche von 130 cm² überführt und durch das Filtertuch Propex 14K filtriert. Die Filtrationstemperatur lag bei 20°C und der Filtrationsdruck bei 2 bar.

Das Filtrat wurde wiederum unter inerten Bedingungen durch eine Filterschicht der Firma PALL vom Typ Seitz ® K 100 mit einer Fläche von 20 cm² filtriert. Das erzeugte Filtrat ergab einen Chlorwert, ermittelt mit Röntgenfluoreszenzanalyse (RFA), von 0,15 Gew. %.

Der Chlorwert konnte somit bei einer Ligandenausbeute von 88,2% um 85% reduziert werden.

### Aufreinigung des Liganden (I), hergestellt nach Variante 1 mittels eines Filtertuches und einer Filterkerze (Vergleichsversuch):

96,9 g des Rohliganden (I), dargestellt nach Variante 1, wurden in 1900 ml entgastem Toluol über Nacht bei 75°C gelöst (Cl-Wert nach Wickbold des Rohliganden (I): 3,5 Gew %). Das aus Cellulose bestehende Filterhilfsmittel DIACEL ® 300 wurde bei 80°C für 14 Stunden in einem Trockenschrank getrocknet. 25 g getrocknetes DIACEL ® 300 wurden in 500 ml getrocknetem Toluol für 2 Stunden eingerührt und danach in ein mit Stickstoff gespültes 2 Liter Druckfiltrationsgerät mit einer Filterfläche von 130 cm² überführt und auf einem Filtertuch anfiltriert. Als Filtertuch wurde eine Propex 14K aus Polypropylen verwendet. Die Filtrationstemperatur lag bei 20°C und der Filtrationsdruck bei 2 bar. Anschließend wurde die Rohlösung langsam und stickstoffüberlagert in das Druckfiltergerät überführt und danach bei 20°C und 2 bar filtriert.

Das Filtrat wurde wiederum unter inerten Bedingungen durch eine 1" Filterkerze der Firma PALL vom Typ Profile ® II mit einer Trenngrenze von 0,3µm filtriert. Von dem erzeugten Filtrat wurde folgender Chlorwert, ermittelt mit Röntgenfluoreszenzanalyse (RFA), bestimmt 0,26 Gew. %.

Das restliche Filtrat wurde abschließend in einem 2L Druckfiltergerät durch eine Ultrafiltrationsmembran der Firma Microdyn Nadir vom Typ UV150T bei 20°C und 2 bar getrennt. Das erzeugte Retentat ergab einen Chlorwert, ermittelt mit Röntgenfluoreszenzanalyse (RFA), von 0,12 Gew. %.

Der Chlorwert konnte somit bei einer Ligandausbeute von 63% um 96,6% reduziert werden.

### Herstellung des Liganden (IV):

Die Organophosphorverbindung 6,6'-[(3,3'-Di-tert-butyl-5,5'-dimethoxy-1,1'-biphenyl-2,2'diyl)bis(oxy)] bis(dibenzo[d,f][1,3,2]dioxaphosphepin mit der Strukturformel (IV) 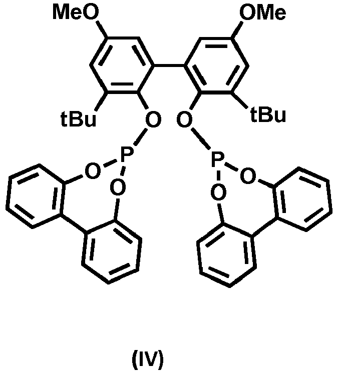 wird auch als Biphephos und nachfolgend als Ligand (IV) bezeichnet.

Seine Herstellung erfolgte analog WO 2012/095253 bzw. WO 2012/095255 mit der Änderung, dass mehrmals mit Lösungsmittel nachgewaschen wurde. Dadurch betrug der Eingangschlorwert 180 ppm.

### Membranreinigung von Ligand (IV):

30 g des Rohliganden (IV) (Cl-Wert bestimmt nach Wickbold des Rohliganden von 180 ppm) wurden in 1900 ml Toluol über Nacht bei 70°C gelöst. Der Ansatz wird in ein mit Stickstoff gespültes 2 Liter Druckfiltrationsgerät mit einer Filterfläche von 130 cm² überführt und durch Ultrafiltrationsmembran der Firma Microdyn Nadir vom Typ UV150T getrennt. Die Membrantemperatur lag bei 20°C und der Transmembrandruck bei 0,6 bar. Der mittlere Permeatfluss während der gesamten Membranreinigung lag bei 15 ml/min.

Das Retentat besaß einen Chlorwert, ermittelt nach Wickbold, von 30 mg/kg.

Der Chlorwert konnte somit bei einer Ligandenausbeute von 32,1g um 83% reduziert werden.

### Membranreinigung von Ligand (IV):

60,5 g des Rohliganden (IV) (Cl-Wert bestimmt nach Wickbold des Rohliganden von 180 ppm) wurden in 1900 ml Toluol über Nacht bei 70°C gelöst. Der Ansatz wird in ein mit Stickstoff gespültes 2 Liter Druckfiltrationsgerät mit einer Filterfläche von 130 cm² überführt und durch Ultrafiltrationsmembran der Firma Microdyn Nadir vom Typ UV150T getrennt. Die Membrantemperatur lag bei 20°C und der Transmembrandruck bei 0,6 bar. Der mittlere Permeatfluss während der gesamten Membranreinigung lag bei 22 ml/min.

Das erzeugte Retentat hatte einen Chlorwert, ermittelt nach Wickbold, von weniger als 10 mg/kg.

Der Chlorwert konnte somit bei einer Ligandenausbeute von 64,4g um 94% reduziert werden.

### Aufreinigung des Liganden (IV) mittels eines PP-Filtertuches in Gegenwart von Cellulose (Vergleichsversuch):

30 g des Rohliganden (IV) (Cl-Wert bestimmt nach Wickbold des Rohliganden von 180 ppm) wurden in 1900 ml Toluol über Nacht bei 70°C gelöst. Das aus Cellulose bestehende Filterhilfsmittel DIACEL ® 300 wurde bei 80°C für 14 Stunden in einem Trockenschrank getrocknet. 20 g getrocknetes DIACEL ® 300 wurden in 500 ml getrocknetem Toluol für 2 Stunden eingerührt und danach in ein mit Stickstoff gespültes 2 Liter Druckfiltrationsgerät mit einer Filterfläche von 130 cm² überführt und auf einem Filtertuch anfiltriert. Als Filtertuch wurde eine Propex 14K aus Polypropylen verwendet. Die Filtrationstemperatur lag bei 20°C und der Filtrationsdruck bei 0,2 bar. Anschließend wurde die Rohlösung langsam und stickstoffüberlagert in das Druckfiltergerät überführt und danach bei 20°C und 0,2 bar filtriert.

Das Filtrat wurde wiederum unter inerten Bedingungen durch eine Filterschicht der Firma PALL vom Typ Seitz ® K 100 mit einer Fläche von 20 cm² filtriert. Das erzeugte Filtrat hatte einen Chlorwert, ermittelt nach Wickbold von 28 mg/kg.

Der Chlorwert konnte somit bei einer Ligandausbeute von 31,2g um 84% reduziert werden.

### Fazit

Die Versuche zeigen, dass es mit den beschriebenen Reinigungsmethoden gelingt, verunreinigte Organophosphorprodukte signifikant von Chlorverbindungen zu befreien. Die so gereinigten Organophophorverbindungen lassen sich bedenkenlos als Liganden in industriell betriebenen Reaktionen einsetzen, da aufgrund ihres geringen Gesamtchlorgehaltes eine Korrosion der stählernen Apparaturen nicht mehr zu befürchten ist. Die betrachteten Organophosphorverbindungen selbst haben sich als Liganden in der homogen katalysierten Hydroformylierung bewährt.

Darüber hinaus lassen sich die hier beschriebenen Reinigungsverfahren gut automatisieren und auf vielerlei Organophosphorprodukte anwenden. Damit ist es dafür prädestiniert, im Rahmen der industriellen Synthese von Organophosphorverbindungen praktiziert zu werden.

## Patentansprüche

1. Verfahren zur Reinigung eines verunreinigten Organophosphorprodukts, welches mindestens eine Organophosphorverbindung, sowie als Verunreinigung mindestens eine Chlorverbindung enthält, mit den folgenden Schritten:
a) Bereitstellen des verunreinigten Organophosphorprodukts;
b) vollständiges Auflösen des verunreinigten Organophosphorprodukts in einem Lösungsmittel unter Erhalt einer verunreinigten Lösung;
c) Trennung der verunreinigten Lösung mittels einer Membrantrenneinheit unter Erhalt einer gereinigten Lösung;
d) Entfernung des Lösungsmittels aus der gereinigten Lösung unter Erhalt eines gereinigten Organophosphorprodukts.

2. Verfahren nach Anspruch 1,
**dadurch gekennzeichnet,**
**dass** sowohl Filtrationsschritte, als auch Membrantrennschritte ausgeführt werden.

3. Verfahren nach Anspruch 1,
**dadurch gekennzeichnet,**
**dass** der Gesamtchlorgehalt des verunreinigten Organophosphorprodukts zwischen 1 000 ppm und 100 000 ppm beträgt, und dass der Gesamtchlorgehalt des gereinigten Organophosphorprodukts zwischen 10 ppm und 10 000 ppm beträgt, vorzugsweise, dass der Gesamtchlorgehalt des gereinigten Organophosphorprodukts zwischen 10 ppm und 1000 ppm beträgt, wobei die Einheit ppm sich auf das Gewicht bezieht.

4. Verfahren nach einem der Ansprüche 1 bis 3,
**dadurch gekennzeichnet,**
**dass** das verunreinigte Organophosphorprodukt mindestens eine Chlorverbindung ausgewählt aus der folgenden Gruppe enthält:
• organische Chloride mindestens ein Kohlenstoffatom enthaltend;
• anorganische Chloride ohne Kohlenstoffatom;
wobei besonders bevorzugt mindestens eine der folgenden Chlorverbindungen enthalten ist:
Phosphortrichlorid, Chlorophosphite, Dichlorophosphite, Hydrochloride von Aminen, Hydrochloride von Alkalimetallen, Chloride von Erdalkalimetallen, chlorhaltige Säuren erhältlich aus der Hydrolyse von Phosphortrichlorid.

5. Verfahren nach einem der vorhergehenden Ansprüche,
**dadurch gekennzeichnet,**
**dass** als Lösungsmittel eine Substanz ausgewählt aus der folgenden Gruppe verwendet wird, oder dass ein Gemisch mehrerer Substanzen dieser Gruppe als Lösungsmittel verwendet wird:
• aromatische Kohlenwasserstoffe wie insbesondere Toluol, Xylol, o-Xylol, Kresol;
• Ether, wie insbesondere Tetrahydrofuran (THF), Petrolether, Diethylether, Methyl-tert-butylether (MTBE);
• Nitrile wie insbesondere Acetonitril (ACN);
• Ethylacetat;
• Aceton;
• Alkohole wie insbesondere Methanol, Ethanol, Isopropanol, Butanol.

6. Verfahren nach Anspruch 5,
**dadurch gekennzeichnet,**
**dass** die verunreinigte Lösung eine einzige organische Phase bildet, insbesondere, dass neben der organischen Phase keine wässrige Phase gebildet wird.

7. Verfahren nach einem der vorhergehenden Ansprüche,
**dadurch gekennzeichnet,**
**dass** das vollständige Auflösen des verunreinigten Organophosphorprodukts in dem Lösungsmittel bei einer Temperatur oberhalb von 20°C und unterhalb der Siedetemperatur des gewählten Lösungsmittels erfolgt, insbesondere bei einer Temperatur von 40°C bis 120°C.

8. Verfahren nach einem der Ansprüche 1 bis 7,
**dadurch gekennzeichnet,**
**dass** die Trennung der verunreinigten Lösung mittels einer Membrantrenneinheit erfolgt, und dass die gereinigte Lösung als Retentat der Membrantrenneinheit anfällt.

9. Verfahren nach Anspruch 8,
**dadurch gekennzeichnet,**
**dass** die Membrantrenneinheit mindestens eine Membran aufweist, deren trennaktives Material aus den folgenden Membranmaterialien ausgewählt ist:
• Silikon basierte Polymere
• Polyethersulfon
• Polyimid

10. Verfahren nach Anspruch 8 oder 9,
**dadurch gekennzeichnet,**
**dass** die Trennung der verunreinigten Lösung mittels der Membrantrenneinheit bei einer Temperatur erfolgt, die von der Temperatur beim vollständigen Auflösen des verunreinigten Organophosphorprodukts in dem Lösungsmittel abweicht, insbesondere, dass die Trennung der verunreinigten Lösung mittels der Membrantrenneinheit bei einer Temperatur von 10 °C bis 80 °C erfolgt.

11. Verfahren nach einem der vorhergehenden Ansprüche,
**dadurch gekennzeichnet,**
**dass** das Organophosphorprodukt mindestens eine Organophosphorverbindung ausgewählt aus der folgenden Gruppe enthält:
• Phosphine;
• Diphosphine;
• Phosphonite;
• Phosphinite;
• bevorzugt Phosphoramidite;
• bevorzugt Monophosphite;
• besonders bevorzugt Bisphosphite, insbesondere ein oder mehrere Bisphosphite der Strukturformeln (I) bis (IV):

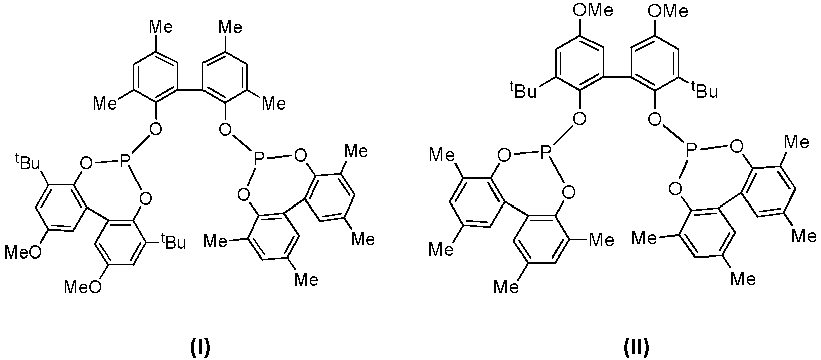

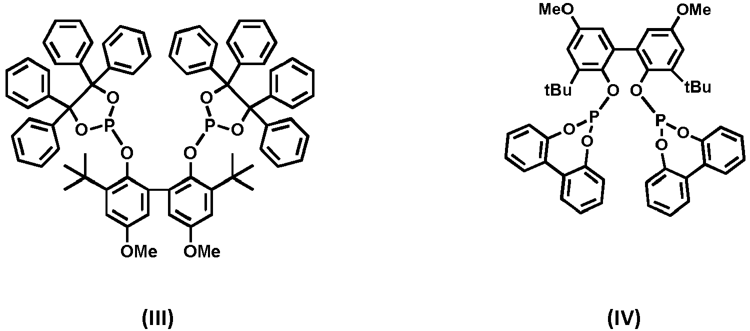

12. Verfahren zur Herstellung eines Katalysatorsystems enthaltend ein Metall sowie mindestens eine Organophosphorverbindung als Ligand,
**dadurch gekennzeichnet,**
**dass** bei der Herstellung des Katalysatorsystems ein gereinigtes Organophosphorprodukt verwendet wird, welches nach Anspruch 11 erhalten wurde.

13. Verfahren zur Hydroformylierung oder zur Hydrocyanierung oder zur Hydrierung von ungesättigten Verbindungen in Gegenwart eines homogenen Katalysatorsystems, welches ein Metall sowie mindestens eine Organophosphorverbindung als Ligand aufweist,
**dadurch gekennzeichnet,**
**dass** das Katalysatorsystem nach Anspruch 12 hergestellt wurde

## Claims

1. Process for the purification of a contaminated organophosphorus product which comprises at least one organophosphorus compound and, as contaminant, at least one chlorine compound, having the following steps:
a) provision of the contaminated organophosphorus product;
b) complete dissolution of the contaminated organophosphorus product in a solvent to give a contaminated solution;
c) separation of the contaminated solution by means of a membrane separation unit to give a purified solution;
d) removal of the solvent from the purified solution to give a purified organophosphorus product.

2. Process according to Claim 1,
**characterized in that**
both filtration steps and membrane separation steps are carried out.

3. Process according to Claim 1,
**characterized in that**
the total chlorine content of the contaminated organophosphorus product is between 1 000 ppm and 100 000 ppm, and that the total chlorine content of the purified organophosphorus product is between 10 ppm and 10 000 ppm, preferably that the total chlorine content of the purified organophosphorus product is between 10 ppm and 1000 ppm, where the unit ppm refers to the weight.

4. Process according to any of Claims 1 to 3,
**characterized in that**
the contaminated organophosphorus product comprises at least one chlorine compound selected from the following group:
• organic chlorides comprising at least one carbon atom;
• inorganic chlorides without carbon atom;
where particularly preferably at least one of the following chlorine compounds is present:
phosphorus trichloride, chlorophosphites, dichlorophosphites, hydrochlorides of amines, hydrochlorides of alkali metals, chlorides of alkaline earth metals, chlorine-containing acids obtainable from the hydrolysis of phosphorus trichloride.

5. Process according to any of the preceding claims,
**characterized in that**
the solvent used is a substance selected from the following group, or that a mixture of several substances from this group is used as solvent:
• aromatic hydrocarbons such as in particular toluene, xylene, o-xylene, cresol;
• ethers, such as in particular tetrahydrofuran (THF), petroleum ether, diethyl ether, methyl tert-butyl ether (MTBE);
• nitriles such as in particular acetonitrile (ACN);
• ethyl acetate;
• acetone;
• alcohols such as in particular methanol, ethanol, isopropanol, butanol.

6. Process according to Claim 5,
**characterized in that**
the contaminated solution forms a single organic phase, in particular, that alongside the organic phase, no aqueous phase is formed.

7. Process according to any of the preceding claims,
**characterized in that**
the complete dissolution of the contaminated organophosphorus product in the solvent takes place at a temperature above 20°C and below the boiling temperature of the selected solvent, in particular at a temperature from 40°C to 120°C.

8. Process according to any of Claims 1 to 7,
**characterized in that**
the separation of the contaminated solution takes place by means of a membrane separation unit, and that the purified solution is produced as retentate of the membrane separation unit.

9. Process according to Claim 8,
**characterized in that**
the membrane separation unit has at least one membrane whose separation-active material is selected from the following membrane materials:
• silicone-based polymers
• polyethersulfone
• polyimide.

10. Process according to Claim 8 or 9,
**characterized in that**
the separation of the contaminated solution takes place by means of the membrane separation unit at a temperature which deviates from the temperature during complete dissolution of the contaminated organophosphorus product in the solvent, in particular that the separation of the contaminated solution takes place by means of the membrane separation unit at a temperature of from 10°C to 80°C.

11. Process according to any of the preceding claims,
**characterized in that**
the organophosphorus product comprises at least one organophosphorus compound selected from the following group:
• phosphines;
• diphosphines;
• phosphonites;
• phosphinites;
• preferably phosphoramidites;
• preferably monophosphites;
• particularly preferably bisphosphites, in particular one or more bisphosphites of the structural formulae (I) to (IV):

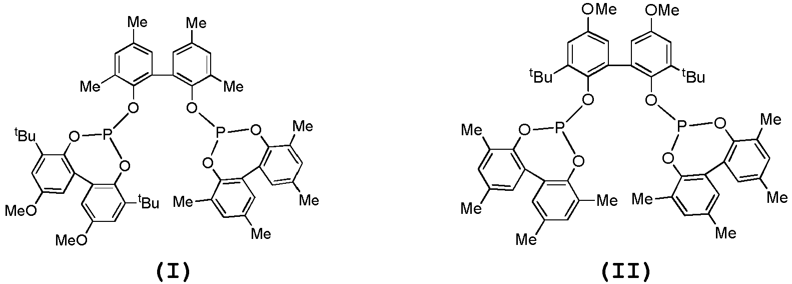

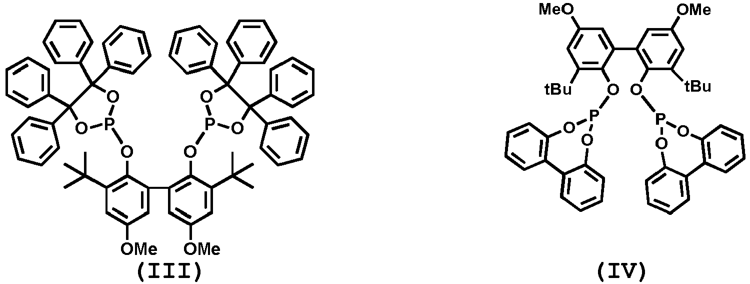

12. Process for producing a catalyst system comprising a metal and at least one organophosphorus compound as ligand,
**characterized in that**
during the production of the catalyst system a purified organophosphorus product is used which has been obtained according to Claim 11.

13. Process for the hydroformylation or for the hydrocyanation or for the hydrogenation of unsaturated compounds in the presence of a homogeneous catalyst system which has a metal and at least one organophosphorus compound as ligand,
**characterized in that**
the catalyst system has been produced according to Claim 12.

## Revendications

1. Procédé pour la purification d'un produit organophosphoré contaminé qui contient au moins un composé organophosphoré, ainsi qu'au moins un composé chloré en tant qu'impureté, comportant les étapes suivantes :
a) disposition du produit organophosphoré contaminé ;
b) dissolution totale du produit organophosphoré contaminé dans un solvant, avec obtention d'une solution contaminée ;
c) fractionnement de la solution contaminée au moyen d'une unité de séparation sur membrane, avec obtention d'une solution purifiée ;
d) élimination du solvant à partir de la solution purifiée, avec obtention d'un produit organophosphoré purifié.

2. Procédé selon la revendication 1,
**caractérisé en ce**
**qu'**on effectue aussi bien des étapes de filtration que des étapes de séparation sur membrane.

3. Procédé selon la revendication 1,
**caractérisé en ce que**
la teneur totale en chlore du produit organophosphoré contaminé est comprise entre 1 000 ppm et 100 000 ppm, et **en ce que** la teneur totale en chlore du produit organophosphoré purifié est comprise entre 10 ppm et 10 000 ppm, de préférence, **en ce que** la teneur totale en chlore du produit organophosphoré purifié est comprise entre 10 ppm et 1 000 ppm, l'unité ppm se rapportant au poids.

4. Procédé selon l'une quelconque des revendications 1 à 3,
**caractérisé en ce que**
le produit organophosphoré contaminé contient au moins un composé chloré choisi dans le groupe suivant :
• des chlorures organiques contenant au moins un atome de carbone ;
• des chlorures inorganiques sans atome de carbone ;
de façon particulièrement préférée au moins l'un des composés chlorés suivants étant contenu :
le trichlorure de phosphore, des chlorophosphites, des dichlorophosphites, des chlorhydrates d'amines, des chlorhydrates de métaux alcalins, des chlorures de métaux alcalino-terreux, des acides chlorés pouvant être obtenus à partir de l'hydrolyse de trichlorure de phosphore.

5. Procédé selon l'une quelconque des revendications précédentes,
**caractérisé en ce**
**qu'**on utilise comme solvant une substance choisie dans le groupe suivant, ou **en ce qu'**on utilise comme solvant un mélange de plusieurs substances de ce groupe :
• des hydrocarbures aromatiques, tels qu'en particulier le toluène, le xylène, l'o-xylène, le crésol ;
• des éthers, tels qu'en particulier le tétrahydrofurane (THF), l'éther de pétrole, l'éther diéthylique, l'oxyde de tert-butyle et de méthyle (MTBE) ;
• des nitriles, tels qu'en particulier l'acétonitrile (ACN) ;
• l'acétate d'éthyle ;
• l'acétone ;
• des alcools, tels qu'en particulier le méthanol, l'éthanol, l'isopropanol, le butanol.

6. Procédé selon la revendication 5,
**caractérisé en ce que**
la solution contaminée forme une unique phase organique, en particulier **en ce qu'**aucune phase aqueuse n'est formée à côté de la phase organique.

7. Procédé selon l'une quelconque des revendications précédentes,
**caractérisé en ce que**
la dissolution totale du produit organophosphoré contaminé dans le solvant a lieu à une température au-dessus de 20 °C et au-dessous de la température d'ébullition du solvant choisi, en particulier à une température de 40 °C à 120 °C.

8. Procédé selon l'une quelconque des revendications 1 à 7,
caractérisé en ce
le fractionnement de la solution contaminée s'effectue au moyen d'une unité de séparation sur membrane, et en ce que la solution purifiée apparaît en tant que rétentat de l'unité de séparation sur membrane.

9. Procédé selon la revendication 8,
**caractérisé en ce que**
l'unité de séparation sur membrane comporte au moins une membrane, dont le matériau actif pour la séparation est choisi parmi les matériaux de membrane suivants :
• des polymères à base de silicone
• la polyéthersulfone
• un polyimide.

10. Procédé selon la revendication 8 ou 9,
**caractérisé en ce que**
le fractionnement de la solution contaminée au moyen de l'unité de séparation sur membrane s'effectue à une température qui diffère de la température lors de la dissolution totale du produit organophosphoré contaminé dans le solvant, en particulier **en ce que** le fractionnement de la solution contaminée au moyen de l'unité de séparation sur membrane s'effectue à une température de 10 °C à 80 °C.

11. Procédé selon l'une quelconque des revendications précédentes,
**caractérisé en ce que**
le produit organophosphoré contient au moins un composé organophosphoré choisi dans le groupe suivant :
• phosphines ;
• diphosphines ;
• phosphonites ;
• phosphinites ;
• de préférence phosphoramidites ;
• de préférence monophosphites ;
• de façon particulièrement préférée bisphosphites, en particulier un ou plusieurs bisphosphites de formules développées (I) à (IV) :

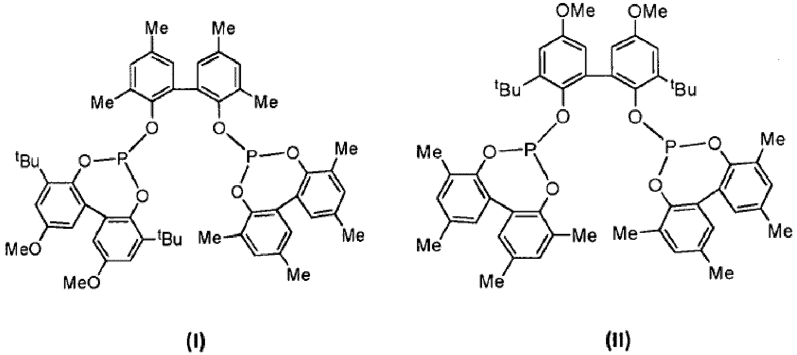

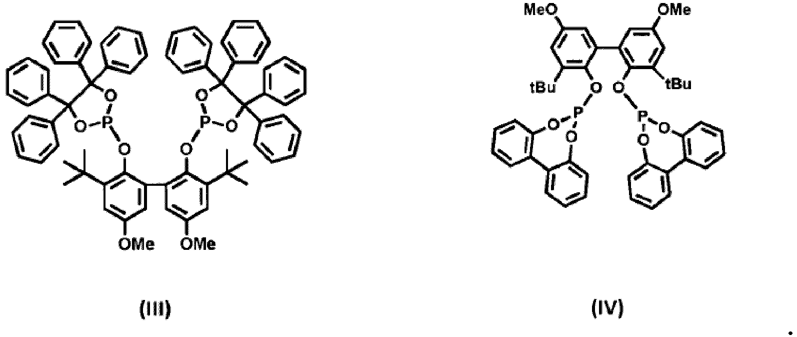

12. Procédé pour la préparation d'un système catalytique contenant un métal ainsi qu'au moins un composé organophosphoré en tant que ligand,
**caractérisé en ce que**
dans la préparation du système catalytique on utilise un produit organophosphoré purifié qui a été obtenu selon la revendication 11.

13. Procédé pour l'hydroformylation ou l'hydrocyanation ou pour l'hydrogénation de composés insaturés, en présence d'un système catalytique homogène qui comporte un métal ainsi qu'au moins un composé organophosphoré en tant que ligand,
**caractérisé en ce que**
le système catalytique a été préparé selon la revendication 12.
